# EUROPEAN PATENT SPECIFICATION

(11) **EP 0 915 123 B1**
(45) Date of publication and mention of the grant of the patent: **09.05.2001**
(21) Application number: 98308835.2
(22) Date of filing: 28.10.1998
(51) Int. Cl.: C08J 3/09, C08G 77/32

(54) **Terminating post cure of silicone elastomers with amino acid esters**
Verfahren zur Beendigung des Nachhärtens von Silikonelastomeren mit Aminosäureestern
Procédé pour la terminaison de la post-réticulation d'élastomères de silicone à l'aide d'esters des aminoacides

(30) Priority: 05.11.1997 US 964547
(43) Date of publication of application: 12.05.1999
(73) Proprietor: DOW CORNING CORPORATION, Midland Michigan 48686-0994 (US)
(72) Inventor: Kadlec, Donald Anthony, Midland, Michigan 48642 (US); Schulz, William James, Jr., Midland, Michigan 48642 (US); Zhang, Shizhong, Midland, Michigan 48642 (US)
(74) Representative: Kyle, Diana

(56) References cited:
- US-A- 5 654 362

## Description

This invention is directed to a method of thickening solvents and, in particular, to a method for the termination of post cure occurring in the thickening of silicone fluids and organic solvents with silicone elastomers.

In US-A 5,654,362, silicone elastomers are used in the thickening of silicone fluids and organic solvents. These elastomers are formed by a hydrosilylation reaction between a multifunctional ≡SiH siloxane and an alkene, preferably α,ω-diene.

According to the above patent, one process that is used in making a silicone elastomer suitable in thickening a low viscosity silicone fluid or organic solvent is:
Step 1 - Gelation $\text{α,ω-diene + ≡Si-H Siloxane + Fluid + Catalyst → Elastomer}$
Step 2 - Shear & Swell $\text{Elastomer + More Fluid → Paste}$

When a crosslinked network is formed in such a reaction, we have determined that steric hindrance of the crosslinked structure prevents the reaction from reaching completion. This is due to the fact that a small amount of residual functionality will remain even after long reaction times and that unreacted functionalities will tend to meet each other when the elastomer is sheared and swollen. We have termed this phenomenon as "post cure". Because residual reactivity causes smooth pasty products to further gel, post cure should be eliminated to maintain a finer appearance and a more flowable rheology of the final paste product.

This is easily achieved, according to this invention, by deactivating the catalyst with an amino acid ester containing post cure terminating agent, most preferably a sulfur containing amino acid ester.

We have discovered that the post cure caused by residual crosslinking hydrosilylation reactions, which typically occur in the preparation of silicone elastomers, are conveniently terminated by introducing a strong platinum group metal complexing ligand to deactivate the catalyst. The aforementioned ligand is preferably an amino acid ester; and most preferably, it is a sulfur containing amino acid ester. In our claimed method, the resulting product is not contaminated by any toxic ingredients. This is an important feature, advantage and benefit, when such products are intended for use in the personal care or health care arenas.

Thus, the present invention provides a method of thickening solvents comprising combining and reacting (A) a ≡Si-H containing polysiloxane of formula R₃SiO(R'₂SiO)ₐ(R''HSiO)_{b}SiR₃ and optionally a ≡Si-H containing polysiloxane of formula HR₂SiO(R'₂SiO)_{c}SiR₂H or a ≡Si-H containing polysiloxane of formula HR₂SiO(R'₂SiO)ₐ(R''HSiO)_{b}SiR₂H where R, R', R'' are alkyl groups of 1-6 carbon atoms; a is 0-250; b is 1-250, and c is 0-250; with (B) an alkene; (C) a platinum group metal catalyst and (D) a solvent; until a silicone elastomer is formed by crosslinking and addition of ≡Si-H across double bonds in said alkene; adding an additional amount of solvent to the silicone elastomer and adding an effective amount of a post cure amino acid ester terminating agent (E); and subjecting the solvent, the post cure terminating agent and the elastomer to shear force until a paste is formed.

Accordingly, our inventive method for thickening solvents involving termination of post cure involves introducing a small amount (one equivalent or more) of a strong platinum group metal complexing ligand, such as an amino acid ester. Especially preferred ligands of our invention are sulfur containing amino acid esters, such as methionine methyl ester CH₃SCH₂CH₂CH(NH₂)COOCH₃, methionine ethyl ester CH₃SCH₂CH₂CH(NH₂)COOC₂H₅, cysteine methyl ester HSCH₂CH(NH₂)COOCH₃, cysteine ethyl ester HSCH₂CH(NH₂)COOC₂H₅ and cystine dimethyl ester {-SCH₂CH(NH₂)COOCH₃}₂.

As a result, we have found chat the catalyst is readily deactivated and that further reaction is impaired in the final product. Our method is not only effective but it is also less hazardous since naturally occurring amino acid derivatives are employed. This is an important feature when the ultimate product is intended for application in the personal care or health care arenas.

Because amine and sulfide functionality are good binding ligands for platinum group metals, amino acid esters have in their free base form amino groups available to complex such catalysts in a hydrosilylation reaction. Even more importantly, however, when the amino acid esters contain sulfur, said sulfide deactivates the catalysts even more effectively.

For our invention, it is preferred that the amino acids not be in zwitterionic form. Rather, they should be derivatized to other forms, such as an ester, so they are more compatible in silicone and organic systems. Additionally, the amino acid derivatives should be in free base form and not in the ammonium salt form; so they are more compatible in silicone and organic systems and they have amino groups available to complex the catalyst. Alternatively, the amino acid derivatives can be converted to a free base form by a simple acid-base reaction after being mixed in the system.

Our invention is useful in any process involving silicone elastomers prepared by a crosslinking reaction between (A) ≡Si-H containing polysiloxanes and (B) an alkene such as an alpha, omega-diene, in the presence of a platinum group metal catalyst (C) and (D) a solvent. Such elastomers are typically swollen with low molecular weight polysiloxanes under a shear force.

Usually, the ≡Si-H containing polysiloxane (A) is a polymer represented by the formula R₃SiO(R'₂SiO)ₐ(R''HSiO)_{b}SiR₃ designated herein as type A¹ and polymers of the formula HR₂SiO(R'₂SiO)_{c}SiR₂H or HR₂SiO(R'₂SiO)ₐ(R''HSiO)_{b}SiR₂H designated herein as type A². In these formulas, R, R' and R", are alkyl groups with 1-6 carbon atoms; a is 0-250; b is 1-250 and c is 0-250. The molar ratio of compounds A²:A¹ is 0-20, preferably 0-5. In preferred embodiments, compounds of types A¹ and A² are used in the reaction; however, it is also possible to conduct the reaction using only compounds of type A¹.

The term alkene (B) includes, and most preferably comprises, an alpha, omega-diene of the formula CH₂=CH(CH₂)ₓCH=CH₂ where x is 1-20. Representative alpha, omega-dienes are 1,4-pentadiene; 1,5-hexadiene; 1,6-heptadiene; 1,7-octadiene; 1,8-nonadiene; 1,9-decadiene; 1,11-dodecadiene; 1,13-tetradecadiene and 1,19-eicosadiene.

The term alkene is also intended to include siloxane monomers or polymers containing two or more terminal alkenyl groups; two or more pendant alkenyl groups; or two or more terminal and pendant groups. One suitable siloxane, for example, is tetramethyldivinyldisiloxane.

These addition and crosslinking reactions require a catalyst (C) to effect reaction between the ≡SiH containing polysiloxane and the alpha, omega-diene. Suitable catalysts are the noble metals and, more particularly, the platinum group metals. Such metal catalysts are more completely described in US Patent 3,923,705. A preferred platinum catalyst is Karstedt's catalyse which is a platinum divinyl tetramethyl disiloxane complex, typically containing one weight percent of platinum, carried in a polydimethylsiloxane fluid or in an organic solvent such as toluene.

Another preferred platinum catalyst is a reaction product of chloroplatinic acid and an organosilicon compound containing terminal aliphatic unsaturation. It is further described in US Patent 3,419,593. These noble metal catalysts are normally used in amounts from 0.00001-0.5 part, per 100 weight parts of the ≡SiH containing polysiloxane. Preferably 0.00001-0.02 part and, most preferably, 0.00001-0.002 part, are used for this invention.

The solvents (D) employed herein include organic compounds and silicone fluids of various types. By "solvent", we mean (i) organic compounds, (ii) compounds containing a silicon atom, (iii) mixtures of organic compounds, (iv) mixtures of compounds containing a silicon atom, or (v) mixtures of organic compounds and compounds containing a silicon atom; used on an industrial scale to dissolve, suspend or change the physical properties of other materials.

The term oil or fluid includes compounds containing a silicone atom, such as (i) low molecular weight linear and cyclic volatile methyl siloxanes, (ii) low molecular weight linear and cyclic volatile and non-volatile alkyl and aryl siloxanes and (iii) low molecular weight linear and cyclic functional siloxanes. Most preferred, however, are low molecular weight linear and cyclic volatile methyl siloxanes (VMS).

VMS compounds correspond to the average unit formula (CH₃)ₐSiO_{(4-a)/2} in which a has an average value of two to three. These compounds contain siloxane units joined by ≡Si-O-Si≡ bonds. Representative units are monofunctional "M" units (CH₃)₃SiO_{1/2} and difunctional "D" units (CH₃)₂SiO_{2/2}.

The presence of trifunctional "T" units CH₃SiO_{3/2} results in the formation of branched linear or cyclic VMS. The presence of tetrafunctional "Q" units SiO_{4/2} results in the formation of branched linear or cyclic VMS.

Linear VMS have the formula (CH₃)₃SiO{(CH₃)₂SiO}_{y}Si(CH₃)₃. The value of y is 0-5. Cyclic VMS have the formula {(CH₃)₂SiO}_{z}. The value of z is 3-6. Preferably, these VMS have a boiling point less than 250°C. and viscosities of 0.65-5.0 centistoke (mm²/s).

Representative linear VMS are hexamethyldisiloxane (MM) with a boiling point of 100°C., viscosity of 0.65 mm²/s and formula Me₃SiOSiMe₃; octamethyltrisiloxane (MDM) with a boiling point of 152°C., viscosity of 1.04 mm²/s and formula Me₃SiOMe₂SiOSiMe₃; decamethyltetrasiloxane (MD₂M) with a boiling point of 194°C., viscosity of 1.53 mm²/s and formula Me₃SiO(Me₂SiO)₂SiMe₃; dodecamethylpentasiloxane (MD₃M) with a boiling point of 229°C., viscosity of 2.06 mm²/s and formula Me₃SiO(Me₂SiO)₃SiMe₃; tetradecamethylhexasiloxane (MD₄M) with a boiling point of 245°C., viscosity of 2.63 mm²/s and formula Me₃SiO(Me₂SiO)₄SiMe₃; and hexadecamethylheptasiloxane (MD₅M) with a boiling point of 270°C., viscosity of 3.24 mm²/s and formula Me₃SiO(Me₂SiO)₅SiMe₃.

Representative cyclic VMS are hexamethylcyclotrisiloxane (D₃) a solid with a boiling point of 134 °C and formula {(Me₂)SiO}₃; octamethylcyclotetrasiloxane (D₄) with a boiling point of 176°C., viscosity of 2.3 mm²/s and formula {(Me₂)SiO}₄; decamethylcyclopentasiloxane (D₅) with a boiling point of 210°C., viscosity of 3.87 mm²/s and formula {(Me₂)SiO}₅; and dodecamethylcyclohexasiloxane (D₆) with a boiling point of 245°C., viscosity of 6.62 mm²/s and formula {(Me₂)SiO}₆.

Representative branched VMS are heptamethyl-3-{(trimethylsilyl)oxy}trisiloxane (M₃T) with a boiling point of 192°C., viscosity of 1.57 mm²/s and formula C₁₀H₃₀O₃Si₄; hexamethyl-3,3,bis{(trimethylsilyl)oxy}trisiloxane (M₄Q) with a boiling point of 222°C., viscosity of 2.86 mm²/s and formula C₁₂H₃₆O₄Si₅; and pentamethyl {(trimethylsilyl)oxy} cyclotrisiloxane (MD₃) with the formula C₈H₂₄O₄Si₄.

Our process also includes the use of low molecular weight linear and cyclic volatile and non-volatile alkyl and aryl siloxanes represented respectively by the formulas R₃SiO(R₂SiO)_{y}SiR₃ and (R₂SiO)_{z}. R is an alkyl group of 1-6 carbon atoms or an aryl group such as phenyl. The value of y is 0-80, preferably 0-20. The value of z is 0-9, preferably 4-6. These polysiloxanes have a viscosity generally in the range of 1-100 centistoke (mm²/s).

Other representative low molecular weight non-volatile polysiloxanes have the general structure: 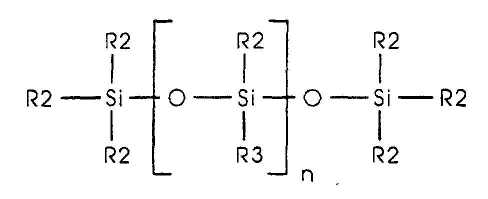 where n has a value to provide polymers with a viscosity in the range of 100-1,000 centistoke (mm²/s).

R2 and R3 are alkyl radicals of 1-20 carbon atoms or an aryl group such as phenyl. Typically, the value of n is about 80-375. Illustrative polysiloxanes are polydimethylsiloxane, polydiethylsiloxane, polymethylethylsiloxane, polymethylphenylsiloxane and polydiphenylsiloxane.

Low molecular weight functional polysiloxanes useful herein are represented by acrylamide functional siloxane fluids, acrylate functional siloxane fluids, amide functional siloxane fluids, amino functional siloxane fluids, carbinol functional siloxane fluids, carboxy functional siloxane fluids, chloroalkyl functional siloxane fluids, epoxy functional siloxane fluids, glycol functional siloxane fluids, ketal functional siloxane fluids, mercapto functional siloxane fluids, methyl ester functional siloxane fluids, perfluoro functional siloxane fluids and silanol functional siloxanes.

Our invention is not limited to swelling silicone elastomers with only low molecular weight polysiloxanes. Other types of solvents, such as organic compounds, can swell the silicone elastomer. Thus, a single solvent or a mixture of solvents may be used.

In general, the organic compounds are aromatic hydrocarbons, aliphatic hydrocarbons, alcohols, aldehydes, ketones, amines, esters, ethers, glycols, glycol ethers, alkyl halides or aromatic halides. Representative of some common organic solvents are alcohols such as methanol, ethanol, 1-propanol, cyclohexanol, benzyl alcohol, 2-octanol, ethylene glycol, propylene glycol and glycerol; aliphatic hydrocarbons such as pentane, cyclohexane, heptane, Varnish Makers and Painters (VM&P) naphtha and mineral spirits; alkyl halides such as chloroform, carbon tetrachloride, perchloroethylene, ethyl chloride and chlorobenzene; amines such as isopropylamine, cyclohexylamine, ethanolamine and diethanolamine; aromatic hydrocarbons such as benzene, toluene, ethylbenzene and xylene; esters such as ethyl acetate, isopropyl acetate, ethyl acetoacetate, amyl acetate, isobutyl isobutyrate and benzyl acetate; ethers such as ethyl ether, n-butyl ether, tetrahydrofuran and 1,4-dioxane; glycol ethers such as ethylene glycol monomethyl ether, ethylene glycol monomethyl ether acetate, diethylene glycol monobutyl ether and propylene glycol monophenyl ether; ketones such as acetone, methyl ethyl ketone, cyclohexanone, diacetone alcohol, methyl amyl ketone and diisobutyl ketone; petroleum hydrocarbons such as mineral oil, gasoline, naphtha, kerosene, gas oil, heavy oil and crude oil; lubricating oils such as spindle oil and turbine oil; and fatty oils such as corn oil, soybean oil, olive oil, rape seed oil, cotton seed oil, sardine oil, herring oil and whale oil.

"Other" miscellaneous organic solvents can also be used, such as acetonitrile, nitromethane, dimethylformamide, propylene oxide, trioctyl phosphate, butyrolactone, furfural, pine oil, turpentine and m-creosol.

Further encompassed by the term solvent are volatile flavoring agents such as oil of wintergreen; peppermint oil; spearmint oil; menthol; vanilla; cinnamon oil; clove oil; bay oil; anise oil; eucalyptus oil; thyme oil; cedar leaf oil; oil of nutmeg; oil of sage; cassia oil; cocoa; licorice; high fructose corn syrup; citrus oils such as lemon, orange, lime and grapefruit; fruit essences such as apple, pear, peach, grape, strawberry, raspberry, cherry, plum, pineapple and apricot; and other useful flavoring agents including aldehydes and esters such as cinnamyl acetate, cinnamaldehyde, eugenyl formate, p-methylanisole, acetaldehyde, benzaldehyde, anisic aldehyde, citral, neral, decanal, vanillin, tolyl aldehyde, 2,6-dimethyloctanal and 2-ethyl butyraldehyde.

In addition, solvent also includes volatile fragrances such as natural products and perfume oils. Some representative natural products and perfume oils are ambergris, benzoin, civet, clove, leaf oil, jasmine, mate', mimosa, musk, myrrh, orris, sandalwood oil and vetivert oil; aroma chemicals such as amyl. salicylate, amyl cinnamic aldehyde, benzyl acetate, citronellol, coumarin, geraniol, isobornyl acetate, ambrette and terpinyl acetate; and the various classic family perfume oils such as the floral bouquet family, the oriental family, the chypre family, the woody family, the citrus family, the canoe family, the leather family, the spice family and the herbal family.

Our basic process comprises in a first step, combining the ≡SiH containing polysiloxane (A), the alkene (B), the solvent (D) and the catalyst (C); and then mixing these ingredients at room temperature until an elastomer is formed.

Additional amounts of silicone fluids or solvents are then added to the elastomer and the resulting mixture is subjected to shear force to form a paste. Any type of mixing and shearing equipment may be used to perform these steps such as a batch mixer, planetary mixer, single or multiple screw extruder, dynamic or static mixer, colloid mill, homogenizer, sonolator or any combination thereof.

Typically, our process uses approximately a 1:1 molar ratio of ≡Si-H containing polysiloxane and alkene, such as alpha, omega-diene. Useful products may also be prepared by conducting the above process with an excess of either the ≡Si-H containing polysiloxane or the alkene but this is a less efficient use of these components. The remainder of our claimed composition comprises silicone fluids or other solvents, in amounts generally within the range of 65-98 percent by weight of the composition and preferably 80-98 percent by weight.

An essential feature of this invention is a second step comprising adding one equivalent or more of a post cure terminating agent (E) to deactivate the platinum catalyst (C), at or during the shear and swell step.

The silicone paste compositions of our invention have particular value in the personal care arena. Because of the unique volatility characteristics of the VMS component of these compositions, they can be used alone or blended with other cosmetic fluids, to form a variety of over-the-counter (OTC) personal care products.

Thus, they are useful as carriers in antiperspirants and deodorants, since they leave a dry. feel and do not cool the skin upon evaporation. They are lubricious and will improve the properties of skin creams, skin care lotions, moisturizers, facial treatments such as acne or wrinkle removers, personal and facial cleansers, bath oils, perfumes, colognes, sachets, sunscreens, pre-shave and after-shave lotions, liquid soaps, shaving soaps and shaving lathers. They can be used in hair shampoos, hair conditioners, hair sprays, mousses, permanents, depilatories and cuticle coats, to enhance gloss and drying time and provide conditioning benefits.

In cosmetics, they will readily function as leveling and spreading agents for pigments in make-ups, color cosmetics, foundations, blushes, lipsticks, lip balms, eyeliners, mascaras, oil removers, color cosmetic removers and powders. They are useful as delivery systems for oil and water soluble substances such as vitamins. When incorporated into sticks, gels, lotions, aerosols and roll-ons, the compositions impart a dry, silky-smooth, payout.

In addition, the claimed compositions exhibit a variety of advantageous and beneficial properties such as clarity, shelf stability and ease of preparation. Hence, they have wide application, but especially in antiperspirants, deodorants, in perfumes as a carrier and for conditioning hair.

Our silicone elastomers, gels and pastes have uses beyond the personal care arena, including their use as a filler or insulation material for electrical cable, a soil or water barrier for in-ground stabilization or as a replacement for epoxy materials used in coil-on-plug designs in the electronics industry.

They are also useful as carrier for crosslinked silicone rubber particles. In that application, (i) they allow ease of incorporation of the particles into such silicone or organic phases as sealants, paints, coatings, greases, adhesives, antifoams and potting compounds; and (ii) they provide for modifying rheological, physical or energy absorbing properties of such phases in either their neat or finished condition.

In addition, our silicone pastes are capable of functioning as carriers for pharmaceuticals, biocides, herbicides, pesticides and other biologically active substances; and can be used to incorporate water and water-soluble substances into hydrophobic systems. Examples of some water-soluble substances are salicylic acid, glycerol, enzymes and glycolic acid.

The following examples further illustrate our invention in more detail.

### Example 1

A gel was prepared using the following ingredients:
(i) 50 g of an ≡SiH siloxane having an average structure represented by Me₃SiO(Me₂SiO)₉₃(MeHSiO)₆SiMe₃
(ii) 260 g of decamethylcyclopentasiloxane (hereafter D5)
(iii) 1.78 g of 1,5-hexadiene, and
(iv) 0.60 g of Karstedt's catalyst with a Pt content of 0.52 %.

Karstedt's catalyst is a preferred platinum catalyst herein and is more fully described in US Patents 3,715,334 and 3,814,730. According to these patents, Karstedt's catalyst is a platinum divinyl tetramethyl disiloxane complex, containing 0.5-1.0 weight percent of platinum, carried in a solvent such as toluene.

A mixture of the above ingredients was stirred in a capped container and heated at 60°C. until gelation. The gel was then heated in a 65-70°C. oven for one hour. The gel was next sheared and swollen with additional D5, to form a silicone paste containing 10 wt % of an elastomer. A solution (0.5 wt % of methionine in methyl ester), 117 mg, was added (as the post cure terminating agent) and mixed with 117 g of the above silicone paste to yield a methionine content of 5 ppm in said paste. The resulting product had a viscosity of 176,000 cP (mPa·s) at 25°C. two hours after it had been prepared. One day later, the paste remained smooth and the viscosity had increased only slightly to 190,000 cP (mPa·s) at 25°C. The viscosity was measured using a Brookfield™ DV-II Viscometer having a TC-type spindle operated at a speed of 2.5 rpm (0.26 rad/s).

### Example 2A - Comparative Example

Another gel was prepared according to Example 1, sheared and swollen in D5 to form a silicone paste containing 10 wt% elastomer. No post cure terminating agent (sulfur-containing amino acid ester) was added. The silicone paste was placed in a jar and gelled to a semisolid after 12 hours.

### Example 2B - Comparative Example

A third gel was prepared according to Example 1, sheared and swollen in D5 to form a silicone paste containing 10 wt% elastomer. Instead of adding a sulfur containing amino acid ester as in Example 1, a solution (0.5 wt% of triphenylphosphine in ethyl acetate) 95 mg, was mixed with 117 g of the silicone paste. The triphenylphosphine content in the silicone paste was 4.1 ppm. The viscosity of the silicone paste two hours after preparation was 290,000 cP (mPa·s). One day later, the silicone paste was a soft gel that had a viscosity of 330,000 cP (mPa·s) at 25°C. The viscosity in this example was measured as in Example 1. This comparative example proves that a similar weight of methionine methyl ester from Example 1 stops post cure more effectively than triphenylphosphine.

## Claims

1. A method of thickening solvents comprising combining and reacting (A) a ≡Si-H containing polysiloxane of formula R₃SiO(R'₂SiO)ₐ(R' 'HSiO)_{b}SiR₃ and optionally a ≡Si-H containing polysiloxane of formula HR₂SiO(R'₂SiO)_{c}SiR₂H or a ≡Si-H containing polysiloxane of formula HR₂SiO(R'₂SiO)ₐ(R' 'HSiO)_{b}SiR₂H where R, R', R'' are alkyl groups of 1-6 carbon atoms; a is 0-250; b is 1-250, and c is 0-250; with (B) an alkene; (C) a platinum group metal catalyst and (D) a solvent; until a silicone elastomer is formed by crosslinking and addition of ≡Si-H across double bonds in said alkene; adding an additional amount of solvent to the silicone elastomer and adding an effective amount of a post cure amino acid ester terminating agent (E) ; and subjecting the solvent, the post cure terminating agent and the elastomer to shear force until a paste is formed.

2. The method of claim 1 wherein said solvent is selected from (i) organic compounds, (ii) compounds containing a silicon atom, (iii) mixtures of organic compounds, (iv) mixtures of compounds containing a silicon atom, and (v) mixtures of organic compounds and compounds containing a silicon atom.

3. The method of claim 1 or 2, in which the post cure terminating agent (E) is methionine methyl ester, methionine ethyl ester, cysteine methyl ester, cysteine ethyl ester or cystine dimethyl ester.

4. A personal care product containing the paste obtained by the method according to any of claims 1 to 3 selected from antiperspirants, deodorants, skin creams, skin care lotions, moisturizers, acne removers, wrinkle removers, facial cleansers, bath oils, perfumes, colognes, sachets, sunscreens, pre-shave lotions, after-shave lotions, liquid soaps, shaving soaps, shaving lathers, hair shampoos, hair conditioners, hair sprays, mousses, permanents, depilatories, cuticle coats, make-ups, color cosmetics, foundations, blushes, lipsticks, lip balms, eyeliners, mascaras, oil removers and cosmetic removers.

5. A product containing the paste obtained by the method according to any of claims 1 to 3 and a material selected from crosslinked silicone rubber particles, pharmaceuticals, biocides, herbicides, pesticides, water and water-soluble substances.

6. Use of the personal care product of claim 4 by applying to the hair, skin or underarm.

7. A method of modifying rheological, physical or energy absorbing properties, of silicone or organic phases selected from sealants, paints, coatings, greases, adhesives, anitfoams and potting compounds, comprising incorporating therein the paste obtained by the method according to any of claims 1 to 3, containing crosslinked silicone rubber particles.

8. A method of filling or insulating an electrical cable comprising incorporating therein the paste obtained by the method according to any of claims 1 to 3.

9. A method of stabilizing in-ground soil or water barriers comprising incorporating into soil the paste obtained by the method according to any of claims 1 to 3.

## Patentansprüche

1. Ein Verfahren zur Verdickung von Lösungsmitteln, umfassend Vereinigen und Umsetzen von (A) einem ≡Si-H-haltigen Polysiloxan der Formel R₃SiO(R'₂SiO)ₐ(R"HSiO)_{b}SiR₃ und fakultativ einem ≡Si-H-haltigen Polysiloxan der Formel HR₂SiO(R'₂SiO)_{c}SiR₂H oder einem ≡Si-H-haltigen Polysiloxan der Formel HR₂SiO(R'₂SiO)ₐ(R"HSiO)_{b}SiR₂H, worin R, R', R" Alkylgruppen mit 1-6 Kohlenstoffatomen sind, a gleich 0-250 ist, b gleich 1-250 ist und c gleich 0-250 ist, mit (B) einem Alken, (C) einem Platingruppenmetallkatalysator und (D) einem Lösungsmittel, bis ein Siliconelastomer durch Vernetzung und Addition von ≡Si-H an Doppelbindungen in dem Alken gebildet wird, Zugeben einer weiteren Menge von Lösungsmittel zu dem Siliconelastomer und Zugeben einer wirksamen Menge eines Aminosäureesters als Mittel zur Beendigung der Nachhärtung (E) und Aussetzen des Lösungsmittels, des Mittels zur Beendigung der Nachhärtung und des Elastomers einer Scherkraft, bis eine Paste gebildet wird.

2. Verfahren nach Anspruch 1, worin das Lösungsmittel ausgewählt ist aus (i) organischen Verbindungen, (ii) Verbindungen mit einem Siliciumatom, (iii) Mischungen von organischen Verbindungen, (iv) Mischungen von Verbindungen, die ein Siliciumatom enthalten, und (v) Mischungen von organischen Verbindungen und Verbindungen, die ein Siliciumatom enthalten.

3. Verfahren nach Anspruch 1 oder 2, in dem das Mittel zur Beendigung der Nachhärtung (E) Methioninmethylester, Methioninethylester, Cysteinmethylester, Cysteinethylester oder Cystindimethylester ist.

4. Körperpflegeprodukt, das die Paste enthält, die nach dem Verfahren nach einem der Ansprüche 1-3 erhalten wird, ausgewählt aus Antitranspirationsmitteln, Deodorantien, Hautcremes, Hautpflegelotionen, Feuchtigkeitscremes, Akneentfernungsmitteln, Antifaltencremes, Gesichtsreinigungsmitteln, Badeölen, Parfums, Erfrischungswässern, Sachets, Sonnenschutzmitteln, Rasierwässern zur Anwendung vor und nach der Rasur, flüssigen Seifen, Rasierseifen, Rasierschäumen, Haarshampoos, Haarkonditionierungsmitteln, Haarsprays, Schäumen, Dauerwellenmitteln, Enthaarungsmitteln, Nagelhautabdeckern, Make-ups, dekorativen Kosmetika, Grundierungen, Rouges, Lippenstiften, Lippenpflegemitteln, Augbrauenstiften, Wimperntuschen, Ölentfernungsmitteln und Make-up-Entfernungsmitteln.

5. Produkt, das die Paste, die nach dem Verfahren gemäss einem der Ansprüche 1-3 erhalten wird, und ein Material, ausgewählt aus vernetzten Siliconkautschukteilchen, pharmazeutischen Substanzen, Bioziden, Herbiziden, Pestiziden, Wasser und wasserlöslichen Substanzen, enthält.

6. Verwendung des Körperpflegeprodukts nach Anspruch 4 durch Auftragung auf das Haar, die Haut oder die Achsel.

7. Verfahren zur Modifikation von rheologischen, physikalischen oder energieabsorbierenden Eigenschaften von Siliconphasen oder organischen Phasen, ausgewählt aus Dichtmitteln, Farben, Beschichtungen, Fetten, Klebstoffen, Antischaummitteln und Einbettmassen, das das darin Einarbeiten der Paste, die durch das Verfahren gemäss einem der Ansprüche 1-3 erhalten wird und die vernetzten Siliconkautschukteilchen enthält, umfasst.

8. Verfahren zum Füllen oder Isolieren eines elektrischen Kabels, das das darin Einfügen der Paste, die durch das Verfahren gemäss einem der Ansprüche 1-3 erhalten wird, umfasst.

9. Verfahren zur Stabilisierung von Boden im Erdreich oder von Wassersperren, umfassend Einarbeitung der Paste, die durch das Verfahren gemäss einem der Ansprüche 1-3 erhalten wird, in den Boden.

## Revendications

1. Procédé pour épaissir des solvants, comprenant la combinaison et la réaction de (A) un polysiloxane contenant ≡Si-H, de formule R₃SiO(R'₂SiO)ₐ(R"HSiO)_{b}SiR₃ et éventuellement un polysiloxane contenant ≡Si-H de formule HR₂SiO(R'₂SiO)_{c}SiR₂H ou un polysiloxane contenant ≡Si-H de formule HR₂SiO(R'₂SiO)ₐ(R"HSiO)_{b}SiR₂H, où R, R', R" sont des groupes alkyle ayant de 1 à 6 atomes de carbone ; a vaut de 0 à 250 ; b vaut de 1 à 250, et c vaut de 0 à 250 ; avec (B) un alcène ; (C) un catalyseur métallique du groupe du platine et (D) un solvant ; jusqu'à ce qu'un élastomère silicone soit formé par réticulation et addition de ≡Si-H sur des doubles liaisons dans ledit alcène ; l'addition d'une quantité supplémentaire de solvant à l'élastomère silicone et l'addition d'une quantité efficace d'un agent de terminaison ester d'acide aminé de post-durcissement (E) ; et soumission du solvant, de l'agent de terminaison de post-durcissement et de l'élastomère à une force de cisaillement jusqu'à ce qu'une pâte soit formée.

2. Procédé selon la revendication 1, dans lequel ledit solvant est choisi parmi (i) les composés organiques, (ii) les composés contenant un atome de silicium, (iii) les mélanges de composés organiques, (iv) les mélanges de composés contenant un atome de silicium, et (v) les mélanges de composés organiques et de composés contenant un atome de silicium.

3. Procédé selon la revendication 1 ou 2, dans lequel l'agent de terminaison de post-durcissement (E) est l'ester méthylique de méthionine, l'ester éthylique de méthionine, l'ester méthylique de cystéine, l'ester éthylique de cystéine ou l'ester diméthylique de cystine.

4. Produit de soin à usage corporel contenant la pâte obtenue par le procédé selon l'une des revendications 1 à 3, choisi parmi les anti-transpirants, les déodorants, les crèmes pour la peau, les lotions pour la peau, les hydratants, les produits anti-acné, les produits antirides, les nettoyants pour le visage, les huiles pour le bain, les parfums, les eaux de Cologne, les sachets parfumés, les écrans solaires, les lotions avant-rasage, les lotions après-rasage, les savons liquides, les savons à barbe, les mousses à raser, les shampooings, les produits de conditionnement des cheveux, les laques pour cheveux, les mousses, les produits pour permanente, les produits dépilatoires, les émollients pour cuticules, les maquillages, les produits cosmétiques teintés, les fonds de teint, les fards, les rouges à lèvres, les baumes à lèvres, les eyeliners, les mascaras, les démaquillants gras et les démaquillants pour maquillage.

5. Produit contenant la pâte obtenue par le procédé selon l'une des revendications 1 à 3 et un matériau choisi parmi les particules de caoutchouc silicone réticulé, les produits pharmaceutiques, les biocides, les herbicides, les pesticides, l'eau et les substances solubles dans l'eau.

6. Utilisation du produit de soin à usage corporel selon la revendication 4 par application sur les cheveux, la peau ou les aisselles.

7. Procédé pour modifier les propriétés rhéologiques, physiques ou d'absorption d'énergie, de phases siliconées ou organiques choisies parmi les matériaux d'étanchéité, les peintures, les revêtements, les graisses, les adhésifs, les anti-moussants et les composés d'empotage, comprenant l'incorporation dans celles-ci de la pâte obtenue par le procédé selon l'une des revendications 1 à 3, contenant des particules de caoutchouc silicone réticulé.

8. Procédé pour remplir ou isoler un câble électrique comprenant l'incorporation dans celui-ci de la pâte obtenue par le procédé selon l'une des revendications 1 à 3.

9. Procédé pour stabiliser des arrêts-barrières à eau ou à sol enterrées comprenant l'incorporation dans le sol de la pâte obtenue par le procédé selon l'une des revendications 1 à 3.
